# EUROPEAN PATENT SPECIFICATION

(11) **EP 4 417 226 B1**
(45) Date of publication and mention of the grant of the patent: **29.04.2026**
(21) Application number: 22879774.2
(22) Date of filing: 03.03.2022
(51) Int. Cl.: A61L 31/00, A61L 31/14

(54) **BIOLOGICAL SLEEVE FOR ACCOMMODATING IMPLANTABLE MEDICAL DEVICE, PREPARATION METHOD THEREFOR AND USE THEREOF**
BIOLOGISCHE HÜLSE ZUR AUFNAHME EINER IMPLANTIERBAREN MEDIZINISCHEN VORRICHTUNG, HERSTELLUNGSVERFAHREN DAFÜR UND VERWENDUNG DAVON
MANCHON BIOLOGIQUE DESTINÉ À RECEVOIR UN DISPOSITIF MÉDICAL IMPLANTABLE, SON PROCÉDÉ DE PRÉPARATION ET SON UTILISATION

(30) Priority: 11.10.2021 CN 202111181226
(43) Date of publication of application: 21.08.2024
(73) Proprietor: Beijing Biosis Healing Biological Technology Co., Ltd., Beijing 102600 (CN)
(72) Inventor: YU, Xueqiao, Beijing 102600 (CN); WU, Wenyue, Beijing 102600 (CN); ZHAO, Bo, Beijing 102600 (CN)
(74) Representative: Perani & Partners S.p.A.
(86) International application number: PCT/CN2022/079071
(87) International publication number: WO 2023/060823

(56) References cited:
- AU-A1- 2006 330 479
- CN-A- 101 361 989
- CN-A- 103 272 278
- CN-A- 107 050 529
- CN-A- 107 050 529
- CN-A- 109 125 922
- CN-A- 109 125 924
- CN-A- 113 908 347
- CN-U- 202 437 991
- US-A1- 2014 275 905

## Description

### TECHNICAL FIELD

The present disclosure belongs to the field of medical apparatus and instruments and biomedical materials. Specifically, the present disclosure relates to a biological sleeve for accommodating an implantable medical device, a preparation method therefor, and a use thereof.

### BACKGROUND

Implantable medical devices are one of the most effective means to treat cardiovascular and cerebrovascular diseases and orthopedic diseases, etc. Of these, Cardiovascular Cardiac Implantable Electronic Device (CIED) including Cardiac Pacemaker (PM), Implantable Cardioverter Defibrillator (ICD) and Cardiac Resynchronization Therapy (CRT) pacemaker and defibrillator, Insertable Cardiac Monitor (ICM), implantable cardiovascular monitors and the like are typical high-risk medical apparatus and instruments. In recent years, new therapeutic devices such as Subcutaneous ICD (S-ICD), leadless pacemakers, and magnetic resonance compatible devices have emerged.

CN107050529 A an intrauterine implant having a pouch structure and comprising a decellularized animal intestinal submucosa matrix material, wherein the pouch structure has an inner cavity and an opening, and the inner cavity is enclosed by the substrate material of the small intestine submucosa. The intrauterine implant comprises a support member, which may be a contraceptive device or an airbag. A method for preparing the intrauterine implant comprises: (1) pretreatment of tissue; (2) inactivation of viruses, wherein a tissue material of a small intestine submucosa is soaked in a peracetic acid-ethanol solution for the inactivation of viruses; (3) washing; (4) decellularization, wherein the decellularization solution is a PBS solution in which trypsin and EDTA are dissolved, and the decellularization process is performed in a multi-frequency ultrasonic device; (5) washing; (6) vacuum freeze-drying and (7) moulding, wherein the substrate material of the small intestine submucosa having been subjected to vacuum freeze-drying that is obtained in step (6) is prepared into the pouch structure.

CIEDs are used mainly for, *e*.*g*. treatment and monitoring of bradyarrhythmias (such as sick sinus syndrome and third-degree atrioventricular block), tachyarrhythmias (such as paroxysmal atrial fibrillation and long Q-T syndrome), and non-cardiac diseases (such as refractory heart failure, neurally mediated syncope, and hypertrophic obstructive cardiomyopathy). In recent years, the number of CIED implantations has shown a gradual upward trend with the increasing morbidity of cardiovascular diseases, the aging of the population, and the expansion of clinical indications.

As a result of the increase in the number of implantations, complications following the CIED implantation are increasingly serious. CIED is composed substantially of a pulse generator, electrodes, and leads. Typically, cardiovascular implants such as a pacemaker and a defibrillator are made of alloy materials (*e.g*. Ni-Co-Cr alloy, Co-Cr-Mo alloy, titanium, and Ti-6A1-4V alloy), stainless steel, and various biocompatible polymer materials, and are generally implanted directly into the cavity formed by the subcutaneous tissue in front of pectoralis major. The main complications occurring in this course include infection, hematoma, ulceration, thrombosis, fistula formation, electrode dislocation, myocardial perforation by electrodes, wear and fracture of leads, *in vivo* displacement of device, etc. Among them, infection, hematoma, and ulceration are common complications after the CIED implantation and have an incidence of up to 7%, which have seriously affected patients and have become important problems in the treatment of cardiovascular diseases.

The occurrence of complications after device implantation is attributed to the following major factors:
(1) the outer casing of the device is often made of a metal or polymer material, which is a foreign matter to the human body and will inevitably cause rejection, thereby causing infection, inflammation, etc.;
(2) for the reasons such as a failure of the size of the implanted device to perfectly fit the cavity or inappropriate limb movement, the implanted device will be caused to move constantly, resulting in friction bleeding, hematoma, thrombus formed by blood clots after ulceration, etc.;
(3) as a result of the occurrence of rejection, inflammation and other diseases after device implantation, the tissue structure may be changed, nearby tissue displaces, and finally fistulization may be caused;
(4) wear and fracture of leads and displacement of device body and the like may all lead to a premature failure of the implanted device, which may incur secondary surgery and more serious complications.

The occurrence of these complications not only increases the economic burden of patients, but also lowers their quality of life, and the mortality even increases especially for those who rely on implantable medical devices such as CIED. Therefore, how to reduce the incidence of infections and the occurrence of complications after device implantation is a major issue that needs to be solved urgently.

### SUMMARY

### Technical Problem

In view of the problems existing in the prior arts, *e*.*g*. susceptibility to complications such as infections after implantation of implantable medical devices in patients, resulting in lowered quality of life and increased mortality of patients, the present disclosure provides a biological sleeve, and an implantable medical device is placed in the accommodation cavity of the biological sleeve, which may avoid direct contact with the human body and reduce the occurrence of complications such as infection and inflammation; moreover, the integrally molded biological sleeve has high tensile strength, which may avoid the risks such as breakage or premature degradation of the sutures that may occur to sutured biological sleeves, and improve the robustness of wrapping the implantable medical device.

### Solution to Problem

In the first aspect, the present disclosure provides a biological sleeve, as defined in claim 1, wherein the biological sleeve is formed of a sterilized and decellularized extracellular matrix material, and has an integrally molded pocket structure; and the pocket structure has an accommodation cavity for accommodating an implantable medical device, and an opening that communicates the accommodation cavity with the outside. The accommodation cavity is enclosed by a first surface body and a second surface body that are opposite to each other; wherein the portion where the first surface body and the second surface body are connected forms a transitional connecting portion of the biological sleeve;
optionally, the transitional connecting portion has a length of 5 to 10 cm along the length direction of the biological sleeve, and the transitional connecting portion has a length of 4 to 8 cm along the width direction of the biological sleeve.

In some embodiments, the biological sleeve according to the present disclosure, wherein at least one microhole is provided on the first surface body and/or the second surface body; optionally, the microhole has a diameter of 1 to 3 mm; optionally, the spacing between the microholes is 10 to 15 mm.

In some embodiments, the biological sleeve according to the present disclosure, wherein the sterilized and decellularized extracellular matrix material is obtained by taking small intestinal submucosa tissue and subjecting it to virus inactivation treatment and decellularization treatment;
preferably, a step of the virus inactivation treatment comprises: immersing the small intestinal submucosa tissue in a virus inactivation solution containing (0.1 to 5)% (v/v) peroxyacetic acid and (5 to 40)% (v/v) ethanol, and treating for 2 to 4 hours at a temperature of 10°C to 40°C;
preferably, a step of the decellularization treatment comprises: immersing the small intestinal submucosa tissue in a decellularization solution containing 0.1 to 2 wt% of trypsin and 0.01 to 0.3 wt% of EDTA, and treating for 10 to 60 min at a temperature of 10°C to 40°C under ultrasonic conditions with ultrasonic power of 5000 W or greater.

In some embodiments, the biological sleeve according to the present disclosure, wherein a step is further included between the step of the virus inactivation treatment and the step of the decellularization treatment, the step being a step of cleaning the small intestinal submucosa tissue until detected conductivity of the small intestinal submucosa tissue is reduced to 10 µS/cm or less;
preferably, a step is further included after the step of the decellularization treatment, the step being a step of cleaning the small intestinal submucosa tissue until the detected conductivity of the small intestinal submucosa tissue is reduced to 1 µS/cm or less.

In the second aspect, the present disclosure provides a method for preparing a biological sleeve according to the first aspect, wherein the preparation method comprises the following steps:
a preparation step of a biofilm layer: preparing a sterilized and decellularized small intestinal submucosa material as a biofilm layer for preparing the biological sleeve;
a coating step of the biofilm layer: alternately coating the biofilm layers on surfaces of both sides of a plate-shaped mold, so that the surfaces of both sides are coated with at least two layers of the biofilm layers, respectively; wherein any one layer of the biofilm layers comprises a coating film layer coated on a partial area of the surface of one side, and an extension film layer that extends continuously to the surface of the other side corresponding to the partial area; and
a freeze-drying step: subjecting the biofilm layer coated on the mold to a freeze-drying treatment in a non-compression environment, so as to integrate the at least two layers of the biofilm layers; wherein the biofilm layers coating two surfaces form a first surface body and a second surface body respectively, the biofilm layer connecting the first surface body and the second surface body forms a transitional connecting portion, and the side of the biofilm layer that does not coat the mold forms an opening, thereby obtaining a biological sleeve integrally molded by the first surface body, the second surface body, and the transitional connecting portion.

In some embodiments, the preparation method according to the present disclosure, wherein the coating step of the biofilm layer comprises:
taking the plate-shaped mold with both sides being a first face and a second face respectively, and reserving top areas of the first face and the second face that are opposite to each other as non-coated areas along the length direction of the mold, and the remaining areas as coated areas;
coating a first biofilm layer on the coated area of the first face, wherein the first biofilm layer forms the coating film layer of the first biofilm layer covering the coated area and the extension film layer of the first biofilm layer extending outward from the coated area; bending the extension film layer in the direction of the second face, so that the extension film layer covers the coated area of the second face; and flattening the extension film layer on the second face to complete the coating step in the direction from the first face to the second face;
coating a second biofilm layer on the coated area of the second face, wherein the second biofilm layer forms the coating film layer of the second biofilm layer covering the coated area and the extension film layer of the second biofilm layer extending outward from the coated area; bending the extension film layer of the second biofilm layer in the direction of the first face, so that the extension film layer of the second biofilm layer covers the coated area of the first face; and flattening the extension film layer on the first face to complete the coating step in the direction from the second face to the first face;
preferably, in the coating step in the direction from the first face to the second face, the extension film layer of the first biofilm layer completely coats the coated area of the second face; and/or the coating step in the direction from the second face to the first face, the extension film layer of the second biofilm layer completely coats the coated area of the first face;
preferably, the coating step in the direction from the first face to the second face, and the coating step in the direction from the second face to the first face are repeated at least once.

In some embodiments, the preparation method according to the present disclosure, wherein the step of the freeze-drying treatment comprises: placing the plate-shaped mold coated with the biofilm layers in a vacuum freeze dryer for non-compression freeze drying;
preferably, conditions for the non-compression freeze drying comprise: pre-freezing to -45°C and holding for 1 to 2 h; then adjusting the temperature to -15°C and holding for 5 to 7 h; readjusting the temperature to 0°C and holding for 2 h; and finally adjusting the temperature to 25°C and holding for 4 h.

In some embodiments, the preparation method according to the present disclosure, wherein the preparation method further comprises the following steps:
a punching step: removing the biological sleeve from the mold, cutting to a desired size, and then punching a hole in the first surface body and/or the second surface body to form at least one microhole on the first surface body and/or the second surface body; preferably, punching a hole in the first surface body and/or the second surface body with microhole spacing of 10 to 15 mm and the diameter of 1 to 3 mm; and
a sterilization step: subjecting the biological sleeve to sterilization treatment with ethylene oxide after heat preservation treatment of the biological sleeve, and then performing aeration to the ethylene oxide to obtain a sterilized biological sleeve; preferably, the heat preservation treatment is carried out for 2 to 4 hours at a temperature of 20°C to 40°C and a humidity of 30% to 70%; preferably, the ethylene oxide is introduced at a concentration of 300 to 1000 mg/L, and the time of the sterilization treatment is 4 to 8 h; preferably, the step of performing aeration to the ethylene oxide is carried out in a ventilated aeration chamber with the temperature controlled at 10°C to 30°C for 14 to 28 days.

In the third aspect, the present disclosure provides an implantable medical apparatus, wherein the implantable medical apparatus comprises:
a biological sleeve according to the first aspect, or a biological sleeve prepared by a method according to the second aspect; and
an implantable medical device that is at least partially placed in an accommodation cavity of the biological sleeve;
optionally, the implantable medical device is selected from the group consisting of devices for diagnosis, monitoring and/or treatment of cardiovascular diseases; optionally, the implantable medical device is any one selected from the group consisting of: a cardiac pacemaker, an implantable cardioverter defibrillator, a cardiac resynchronization therapy pacemaker, an implantable defibrillator, an insertable cardiac monitor, and an implantable cardiovascular monitor.

In some embodiments, use of a biological sleeve according to the present disclosure or a biological sleeve prepared by a method according to the present disclosure in accommodation of an implantable medical device;
optionally, the implantable medical device is selected from the group consisting of devices for diagnosis, monitoring and/or treatment of cardiovascular diseases; optionally, the implantable medical device is any one selected from the group consisting of: a cardiac pacemaker, an implantable cardioverter defibrillator, a cardiac resynchronization therapy pacemaker, an implantable defibrillator, an insertable cardiac monitor, and an implantable cardiovascular monitor.

### Advantageous Effects of the Invention

In some embodiments, the present disclosure provides a biological sleeve, in which an implantable medical device is placed in the accommodation cavity thereof that may provide a physical barrier for the implantable medical device to avoid direct contact of the implantable medical device with the body, reducing the occurrence of complications such as infection, inflammation, scarring, and calcification after implantation. Furthermore, the integrally molded structure of the biological sleeve offers high tensile strength, which can avoid poor service conditions of sutured biological sleeves, such as breakage or premature degradation of sutures, and realize stable physical isolation of the implantable device.

In some embodiments, the biological sleeve provided in the present disclosure is formed from a sterilized and decellularized small intestinal submucosa material, which has high safety in use and a good tissue repair capacity, can promote tissue repair and healing at the implantation site, and accelerate postoperative recovery after implantation.

Further, the small intestinal submucosa material can be degraded under the action of enzymes *in vivo,* and ultimately the implantable medical device is externally wrapped by the new tissue to form a cystic structure, which further reinforces the implantable medical device, maintains the normal operation of the device, and prolongs the service life of the device.

In some embodiments, the biological sleeve provided in the present disclosure releases antibacterial peptides after degradation, thereby reducing the probability for occurrence of complications such as inflammation and infection after implantation of medical devices.

In some embodiments, the method for preparing a biological sleeve provided in the present disclosure may allow for preparation of an integrally molded biological sleeve and dispense with suturing of the sleeve body using sutures, which can reduce the preparation time and preparation cost of the biological sleeve, and avoid the risk of the suture breakage and the risk of the sutures degrading earlier than the sleeve.

### BRIEF DESCRIPTION OF THE DRAWINGS

FIG. 1 shows the schematic diagram for the structure of a biological sleeve.
FIG. 2 shows the schematic diagram for the structure of a plate-shaped mold.
FIG. 3 shows the schematic diagram for the structure of a biofilm layer.
FIG. 4-A shows the schematic diagram for the process of coating a biofilm layer from the first face to the second face.
FIG. 4-B shows the schematic diagram for the process of coating a biofilm layer from the second face to the first face.
FIG. 5 shows the real picture of a biological sleeve, in which A is a schematic diagram for one side of the biological sleeve, B is a schematic diagram for the other side of the biological sleeve, C is a schematic diagram for the pocket opening of the biological sleeve, and D is a lateral oblique view of the biological sleeve.
FIG. 6 shows a statistical chart of the tensile strength of four samples, in which sample 1 is dry-integrated SIS biological sleeve, sample 2 is dry-suture SIS biological sleeve, sample 3 is hydration-integrated SIS biological sleeve, and sample 4 is hydration- suture SIS biological sleeve.

Description of Reference Numerals:
1-pocket structure, 11-first surface body, 12-second surface body, 13-opening, 14-transitional connecting portion, 141-first transitional connecting portion, 142-second transitional connecting portion, 143-third transitional connecting portion;
2-mold, 21-first face, 211-first non-coated area, 212-first coated area, 22-second face, 221-second non-coated area, 222-second coated areas;
3-biofilm layer, 31-coating film layer, 32-extension film layer, 321-first extension film layer, 322-second extension film layer, 323-third extension film layer.

### DETAILED DESCRIPTION

Various exemplary examples, features and aspects of the present disclosure will be described in detail below. Herein the specific term "exemplary" means "used as an instance or example or explanatory". An "exemplary" example given here is not necessarily construed as being superior to or better than other examples.

In addition, numerous details are given in the following specific embodiments for the purpose of better explaining the present disclosure. It should be understood by a person skilled in the art that the present disclosure can still be realized even without some of those details. In some of the examples, methods, means, apparatus and instruments, and steps that are well known to a person skilled in the art are not described in detail so that the principle of the present disclosure becomes apparent.

Unless otherwise stated, the units used in the present specification are all international standard units, and the numerical values and numerical ranges used in the present disclosure should all be construed as including inevitable systematic errors in industrial production.

In the present specification, the term "may" used herein involves both the meaning of doing something and the meaning of not doing something.

Phrases such as "some specific/preferred embodiments", "other specific/preferred embodiments", and "embodiments" referred to in the present specification mean that particular elements (for example, features, structures, properties and/or characteristics) described in relation to this embodiment are included in at least one of the embodiments described herein, and may or may not exist in other embodiments. Additionally, it should be understood that the elements may be combined in any suitable manner into various embodiments.

In the present specification, the numerical range represented by "numerical value A to numerical value B" refers to the range including the endpoint values A and B.

In the present specification, "v/v" is used to represent volume percentage content, "wt%" is used to represent mass percentage content, and "w/v" represents a concentration in "g/mL".

In the present specification, when "normal temperature" or "room temperature" is used, the temperature may be 10°C to 40°C.

### Biological Sleeve

According to the first aspect of the present disclosure, there is provided a biological sleeve. The biological sleeve is formed of a sterilized and decellularized extracellular matrix material, and has an integrally molded pocket structure 1. The pocket structure 1 has an accommodation cavity for accommodating an implantable medical device, and an opening 13 that communicates the accommodation cavity with the outside.

By placing the implantable medical device in the accommodation cavity of the biological sleeve, it is possible to provide a layer of physical barrier for the medical device, reduce the incidence of complications such as infection caused by direct contact of the metal or polymer material of the outer casing of the device with the human body, and reduce device failure or damage caused by displacement of the device, etc. Although biological sleeves can act as a physical barrier, currently available biological sleeves for accommodating implantable medical devices are mostly sutured to form a pocket-like structure, and the suturing process needs to be controlled in a sterile environment, which complicates the processing of the biological sleeves and increases the processing cost and processing time of the biological sleeves. Besides, risks such as suture breakage or suture degradation earlier than the degradation of the sleeve body may occur to the biological sleeves formed by means of suturing during use, which brings about an adverse effect to the *in vivo* implantation of the medical device. In contrast, the biological sleeve of the present disclosure has an integrally molded pocket structure 1, which does not need to be sutured with sutures and effectively reduces adverse consequences such as separation or displacement of the pocket body as a result of breakage or premature degradation of sutures, etc. Furthermore, compared with the sutured biological sleeves, the integrally molded biological sleeves of the present disclosure have higher tensile strength and increased use intensity and robustness, which may further reduce the probability of complications after device implantation.

In some embodiments, the accommodation cavity of the biological sleeve is enclosed by a first surface body 11 and a second surface body 12. The portion where the first surface body 11 and the second surface body 12 are connected forms a transitional connecting portion 14 of the biological sleeve.

In the present disclosure, the first surface body 11 and the second surface body 12 may have a variety of different shapes and sizes. Exemplarily, the first surface body 11 and the second surface body 12 may be triangular, quadrilateral, pentagonal, circular or the like, respectively. Concretely, the first surface body 11 and the second surface body 12 are configured to enclose an accommodation cavity for accommodating an implantable medical device. The shapes and sizes of the first surface body 11 and the second surface body 12 may be adapted according to the shape and size of the implantable medical device.

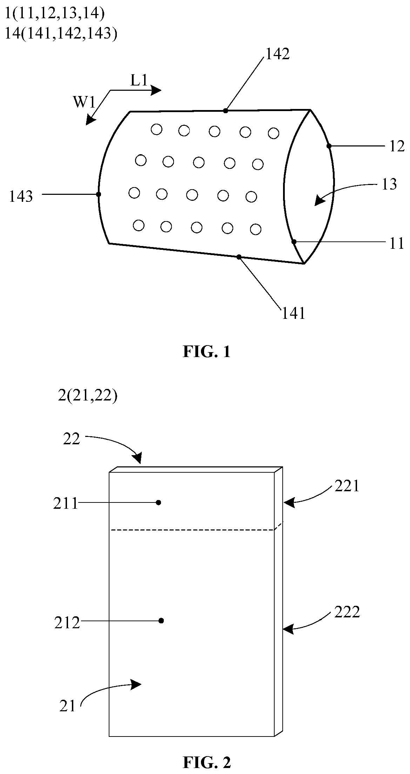

In some embodiments, as shown in FIG. 1, the opposite surfaces of the first surface body 11 and the second surface body 12 are quadrilateral, and the first surface body 11 and the second surface body 12 have two sides extending in the length direction (L1 direction) of the biological sleeve and two sides extending in the width direction (W1 direction) of the biological sleeve, respectively.

In some embodiments, either of the first surface body 11 and the second surface body 12 has a length of 5 to 10 cm and a width of 4 to 8 cm. Exemplarily, the area of either of the first surface body 11 and the second surface body 12 is configured to be 5.4×5, 6.9×6.5, 6.9×8 or 6.9×9.5 (width × height, unit: cm).

In some embodiments, the first surface body 11 and the second surface body 12 are opposite to each other and have the same shape and size. In other embodiments, the first surface body 11 and the second surface body 12 may also be opposite but have different shapes or sizes.

In the present disclosure, the transitional connecting portion 14 is formed on the sides where the first surface body 11 and the second surface body 12 partially are connected, such that the first surface body 11 and the second surface body 12 enclose an accommodation cavity suitable for accommodating an implantable medical device. An opening 13 is formed between the sides of the first surface body 11 and the second surface body 12 that are not connected, and an implantable medical device may be partially or wholly placed in the accommodation cavity through the opening 13.

In the present disclosure, the opening 13 communicates the accommodation cavity of the biological sleeve with the outside. The term "outside" refers to the space out of the accommodation cavity of the biological sleeve, which may be either internal or external environment, depending upon the environment in which the biological sleeve is used.

In some embodiments, as shown in FIG. 1, the first surface body 11 and the second surface body 12 are a quadrangle extending in the length direction (L1 direction) and the width direction (W1 direction) of the biological sleeve, respectively. The transitional connecting portion 14 is formed among any three opposite sides of the first surface body 11 and the second surface body 12. An opening 13 is formed between the sides of the first surface body 11 and the second surface body 12 that are not connected.

It is to be noted that the transitional connecting portion 14 of the present disclosure is formed after the first surface body 11 and the second surface body 12 are joined, and does not affect the integrally molded structure of the biological sleeve. Moreover, the transitional connecting portion 14 serves as a joint portion of the first surface body 11 and the second surface body 12 as long as it allows the first surface body 11 and the second surface body 12 to be joined in an integrally molded structure in the length direction (L1 direction) and the width direction (W1 direction) of the biological sleeve. As such, the present disclosure sets only the lengths of the transitional connecting portion 14 in the L1 direction and in the W1 direction, while the cross section of the transitional connecting portion 14 in the L1 direction or in the W1 direction may be in any shape, *e.g.* a polygon, a circle, or even a dot shape.

In some embodiments, the transitional connecting portion 14 is formed on both sides of the first surface body 11 and the second surface body 12 extending in the width direction (W1 direction) and on one side extending in the length direction (L1 direction), such that the first surface body 11 and the second surface body 12 enclose a pocket structure 1 having an accommodation cavity. More concretely, the transitional connecting portion 14 includes a first transitional connecting portion 141 and a second transitional connecting portion 142 on both sides in the width direction (W1 direction), and a third transitional connecting portion 143 in the length direction (L1 direction) that is connected with the opposite ends of the first transitional connecting portion 141 and the second transitional connecting portion 142, such that the first surface body 11 and the second surface body 12 enclose an integrally molded pocket structure 1 having an accommodation cavity. An opening 13 is formed on the other side opposite to the third transitional connecting portion 143 in the length direction.

In other embodiments, the first transitional connecting portion 141 and the second transitional connecting portion 142 may also be on two opposite sides in the length direction (L1 direction); the third transitional connecting portion 143 is on one side in the width direction (W1 direction); and an opening 13 is formed on the other side opposite to the third transitional connecting portion 143 in the width direction (W1 direction).

In the present disclosure, the biological sleeve is formed of a sterilized and decellularized Extracellular matrix (ECM) material.

The sterilized and decellularized ECM material has high biocompatibility, low immunogenicity, and high antibacterial properties. It is suitable for encapsulating an implantable medical device to be implanted *in vivo,* which plays the roles in bacteria resistance and wound isolation, avoiding direct contact of the device with the body, decreasing device displacement, etc., thereby effectively reducing occurrence of inflammation, infection, calcification, and scarring.

Further, the ECM material has a three-dimensional tissue structure, which can promote the migration and growth of cells at the implantation site, promote the growth of blood vessels, and promote the growth of new tissue. Moreover, the ECM material is a degradable biomaterial and will degrade after being implanted in the body for a period of time. Then the new tissue wraps the implanted device to form a cystic structure, which further stabilizes the implanted medical device, reduces the occurrence of various complications such as infection, ulceration, and hematoma, while ensuring the normal operation of the medical device and extending the service life of the device.

In some embodiments, the sterilized and decellularized ECM material is a sterilized and decellularized small intestinal submucosa material. The Small Intestinal Submucosa (SIS) material has excellent capacities of promoting tissue repair and healing.

In some embodiments, at least one microhole is provided on the first surface body 11 and/or the second surface body 12. By providing the microhole, on the one hand, it can prevent the body fluids from being accumulated inside the pocket; on the other hand, it enables the new blood vessels and tissue at the implantation site to be effectively combined with the biological sleeve and wrap the implanted medical device to form a cystic structure, thus playing the role in stabilizing the device. In some preferred embodiments, the diameter of the microhole is 1 to 3 mm and the spacing between the microholes is 10 to 15 mm.

In some embodiments, the biological sleeve releases antibacterial peptides after degradation, and exerts the antibacterial and anti-infective effects. Further, the antibacterial peptide is one or more selected from the group consisting of NK-lysin, LL-37, and PR-39. By releasing abundant antibacterial peptides, the risk of infection caused by the implantable medical device *in vivo* may be reduced.

The biological sleeve of the present disclosure provides, through the integrally molded first surface body 11, second surface body 12 and transitional connecting portion 14, a natural, low-immunogenic, and antibacterial material barrier for the implantable medical device to prevent the metal or polymer material of the outer casing of the device from being directly in contact with the body. Meanwhile, the biological sleeve does not need to be sutured with sutures, so its processing cost is low and its processing technology is simple. Moreover, compared with the sutured biological sleeves, the integrally molded biological sleeves of the present disclosure have higher tensile strength and can avoid the risks incurred by breakage or premature degradation of sutures, and its service performance is further improved.

### Sterilized and Decellularized Extracellular Matrix Material

The sterilized and decellularized extracellular matrix material of the present disclosure is a sterilized and decellularized small intestinal submucosa material obtained by taking small intestinal submucosa tissue and subjecting it to virus inactivation treatment and decellularization treatment. The material has the advantages of high biocompatibility, low immunogenicity, good antibacterial properties and so forth, and is effective in promoting tissue repair and healing.

In some embodiments, the small intestinal submucosa tissue is obtained by removing the lymphoid tissue, then rinsing until the surface is free of stains, and draining water. In some specific embodiments, the small intestinal submucosa tissue is derived from porcine small intestinal submucosa tissue. In some specific embodiments, the small intestinal submucosa tissue is cut to a prescribed size, *e.g.* the cut small intestinal submucosa tissue has a length of 2 to 20 cm and a width of 2 to 10 cm.

In some embodiments, the step of the virus inactivation treatment comprises: immersing small intestinal submucosa tissue in a virus inactivation solution containing peroxyacetic acid and ethanol for virus inactivation. The concentration of peroxyacetic acid in the virus inactivation solution is (0.1 to 5)% (v/v). Exemplarily, the concentration of peroxyacetic acid is 0.5% (v/v), 1% (v/v), 2% (v/v), 3% (v/v), 4% (v/v), etc. The concentration of ethanol in the virus inactivation solution is (5 to 40)% (v/v). Exemplarily, the concentration of ethanol is 8% (v/v), 10% (v/v), 15% (v/v), 20% (v/v), 25% (v/v), 30% (v/v), 35% (v/v), etc.

In some specific embodiments, the time of virus inactivation treatment is 2 to 4 hours, and the temperature is 10°C to 40°C. In some specific embodiments, the volume ratio of the virus inactivation solution to the small intestinal submucosa tissue is (20 to 40):1, *e.g.* 25:1, 30:1, 35:1, or 38:1.

Through the virus inactivation treatment, harmful components in the small intestinal submucosa tissue are effectively removed to ensure its biosafety in the preparation of a biological sleeve.

In some embodiments, the step of the decellularization treatment comprises: immersing small intestinal submucosa tissue in a decellularization solution containing trypsin and EDTA for decellularization treatment. In the decellularization solution, the trypsin content is 0.1 to 2 wt%. Exemplarily, the trypsin content is 0.5 wt%, 0.8 wt%, 1 wt%, 1.2 wt%, 1.5 wt%, 1.8 wt%, etc. In the decellularization solution, the EDTA content is 0.01 to 0.3 wt%. Exemplarily, the EDTA content is 0.05 wt%, 0.08 wt%, 0.1 wt%, 0.15 wt%, 0.2 wt%, 0.22 wt%, 0.25 wt%, 0.28 wt%, etc.

Further, the decellularization solution is a PBS buffer containing trypsin and EDTA and has a pH of 6 to 8. In some specific embodiments, the decellularization treatment is carried out under ultrasonic conditions. Further, the small intestinal submucosa tissue is put in an ultrasonic cleaner for decellularization treatment.

Preferably, during the decellularization treatment, the ultrasonic power is 5000 W or greater, the temperature is 10°C to 40°C, and the ultrasonic time is 10 to 60 min.

Through the decellularization treatment, it is possible to effectively remove the immunogenic components such as nucleic acids, cell membranes, and nuclear fragments from the small intestinal submucosa tissue to decrease the immunogenicity of the material, such that the biological sleeve prepared therefrom is suitable for implantation into the body.

In some embodiments, a step is further included between the step of the virus inactivation treatment and the step of the decellularization treatment, the step being a primary cleaning step of cleaning the small intestinal submucosa tissue until the detected conductivity of the small intestinal submucosa tissue is reduced to 10 µS/cm or less.

Further, the cleaning solution for the primary cleaning of the small intestinal submucosa tissue is a PBS buffer at pH 6-8. The temperature of the cleaning solution is 10°C to 40°C. The volume ratio of the cleaning solution to the small intestinal submucosa tissue is (20 to 40):1, *e.g.* 25:1, 30:1, 35:1, or 38:1.

In some specific embodiments, the primary cleaning step is carried out in an ultrasonic cleaner.

In some specific embodiments, the small intestinal submucosa is cleaned 2 to 4 times with a cleaning solution, 10 to 30 min for each time; and then cleaned with water for injection at 10°C to 40°C at a volume ratio of the water for injection to the small intestinal submucosa tissue of (20 to 40):1 until the detected conductivity is 10 µS/cm or less.

In the present disclosure, the detected conductivity refers to a difference in conductivities between the water for injection after cleaning the small intestinal submucosa tissue and the water for injection before cleaning the small intestinal submucosa tissue.

Through the primary cleaning step, the detected conductivity of the small intestinal submucosa tissue is reduced to 10 µS/cm or less to eliminate the residues of the substances from the treatment solution.

In some embodiments, after the step of the decellularization treatment, there is further included a secondary cleaning step of cleaning the small intestinal submucosa tissue until the detected conductivity of the small intestinal submucosa tissue is reduced to 1 µS/cm or less.

Further, the cleaning solution for the secondary cleaning of the small intestinal submucosa tissue is a PBS buffer at pH 6-8. The temperature of the cleaning solution is 10°C to 40°C. The volume ratio of the cleaning solution to the small intestinal submucosa tissue is (20 to 40):1, *e.g.* 25:1, 30:1, 35:1, or 38:1.

In some specific embodiments, the small intestinal submucosa is cleaned 2 to 4 times with a cleaning solution, 10 to 30 min for each time; and then cleaned with water for injection at 10°C to 40°C at a volume ratio of the water for injection to the small intestinal submucosa tissue of (20 to 40):1 until the detected conductivity is 1 µS/cm or less.

Through the secondary cleaning step, the detected conductivity of the small intestinal submucosa tissue is reduced to 1 µS/cm or less to eliminate the residues of substances from the treatment solution.

### Implantable Medical Apparatus

The implantable medical apparatus provided in the present disclosure comprises the biological sleeve provided in the present disclosure, and an implantable medical device that is at least partially placed in the accommodation cavity of the biological sleeve.

The biological sleeve of the present disclosure provides a layer of safe and stable physical barrier for the device implanted in the body, and can avoid occurrence of various complications caused by direct contact of the device with the body. Besides, the integrally molded structure of the biological sleeve allows it to have high tensile strength, which avoids labile factors such as breakage or premature degradation of sutures possibly occurring to sutured biological sleeves, and has great prospect of clinical applications.

In some optional embodiments, the implantable medical device is selected from the group consisting of devices for diagnosis, monitoring and/or treatment of cardiovascular diseases. In some preferred embodiments, the implantable medical device is any one selected from the group consisting of: a cardiac pacemaker, an implantable cardioverter defibrillator, a cardiac resynchronization therapy pacemaker, an implantable defibrillator, an insertable cardiac monitor, an implantable cardiovascular monitor, etc.

### Preparation Method for Biological Sleeve

In the present disclosure, the preparation method for the biological sleeve comprises the following steps:
a preparation step of a biofilm layer 3: forming the biofilm layer 3 from a sterilized and decellularized extracellular matrix material;
a coating step of the biofilm layer 3: alternately coating the biofilm layers 3 on the surfaces of both sides of a plate-shaped mold 2, so that the surfaces of both sides are coated with at least two layers of the biofilm layers 3, respectively; wherein any one layer of the biofilm layers 3 comprises a coating film layer 31 coated on a partial area of the surface of one side, and an extension film layer 32 that extends continuously to the surface of the other side corresponding to the partial area; and
a freeze-drying step: subjecting the biofilm layer 3 coated on the mold 2 to a freeze-drying treatment in a non-compression environment, so as to integrate the at least two layers of the biofilm layers 3; wherein the biofilm layers 3 coating two surfaces form a first surface body 11 and a second surface body 12 respectively, the biofilm layer 3 connecting the first surface body and the second surface body forms a transitional connecting portion 14, and the side of the biofilm layer 3 that does not coat the mold 2 forms an opening 13, thereby obtaining the biological sleeve.

The preparation method for the biological sleeve provided in the present disclosure may be used to prepare an integrally molded biological sleeve with high tensile strength. In the preparation process, the sleeve body does not need to be sutured with sutures, which simplifies the preparation steps of the biological sleeve, shortens the preparation time, and reduces the preparation cost.

In some embodiments, the biofilm layer 3 is a biofilm layer 3 with a prescribed size that is formed of a sterilized and decellularized extracellular matrix material. Further, the biofilm layer 3 is formed of a sterilized and decellularized small intestinal submucosa material.

In some embodiments, the coating step of biofilm layer 3 comprises S1 to S4:
S1: taking a plate-shaped mold 2 with both sides being a first face 21 and a second face 22, respectively; as shown in FIG. 2, reserving the top areas of the first face 21 and the second face 22 that are opposite to each other as non-coated areas along the length direction of the mold 2, and the remaining areas as coated areas. For ease of illustration, the non-coated area of the first face 21 is referred to as the first non-coated area 211, and the non-coated area of the second face 22 opposite to the non-coated area of the first face 21 is referred to as the second non-coated area 221; the coated area of the first face 21 is referred to as the first coated area 212, and the coated area of the second face 22 opposite to the coated area of the first face 21 is referred to as the second coated area 222.

In some specific embodiments, the plate-shaped mold 2 is a stainless steel baseplate. In some specific embodiments, the size (width × height, unit: cm) of either of the first face 21 and the second face 22 is 5.4×7; 6.9×8.5; 6.9×10; 6.9× 11.5, etc.

S2: coating the biofilm layer 3 on the first coated area 212 of the first face 21, wherein the biofilm layer 3 forms a coating film layer 31 covering the first coated area 212 and an extension film layer 32 extending outward from the first coated area 212; bending the extension film layer 32 in the direction of the second face 22, so that the extension film layer 32 covers the second coated area 222 of the second face 22; and flattening the extension film layer 32 on the second face 22, so that the first face 21 and the second face 22 are coated with a layer of the biofilm layer 3 respectively, thereby completing the coating step in the direction from the first face 21 to the second face 22.

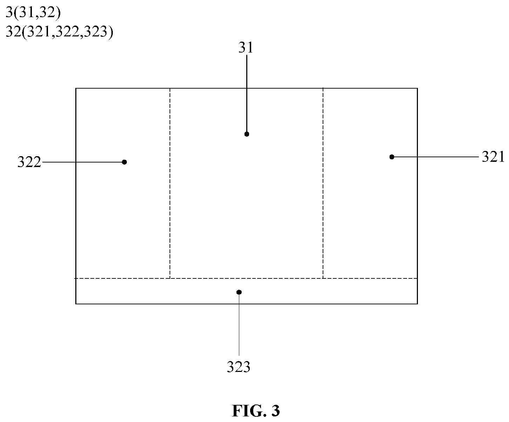

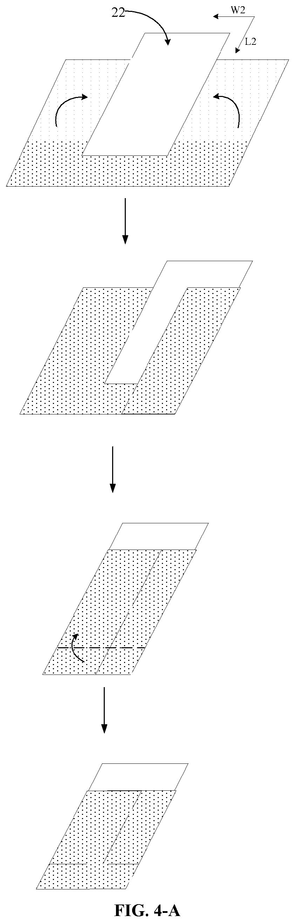

Specifically, as shown in FIG. 4-A, the biofilm layer 3 is taken to coat the first coated area 212 of the first face 21, where the portion that the biofilm layer 3 covers the first coated area 212 serves as the coating film layer 31. Besides, the biofilm layer 3 has, in both the length direction (L2 direction) and the width direction (W2 direction) of the mold 2, a portion extending outward and beyond the first coated area 212, and this portion is the extension film layer 32. As shown in FIG. 3, the extension film layer 32 includes a first extension film layer 321 and a second extension film layer 322 on two opposite sides in the width direction of the mold 2, and a third extension film layer 323 that connects the first extension film layer 321 and the second extension film layer 322.

The extension film layer 32 is bent in the direction of the second face 22. Specifically, as shown in FIG. 4-A, by folding three times, the first extension film layer 321, the second extension film layer 322, and the third extension film layer 323 are all coated on the second coated area 222 of the second face 22. Exemplarily, firstly, the first extension film layer 321 and the portion of the third extension film layer 323 connecting the first extension film layer 321 are folded towards the second face 22, such that the first extension film layer 321 is coated on the second coated area 222; and thereafter, the second extension film layer 322 and the portion of the third extension film layer 323 connecting the second extension film layer 322 are folded towards the second face 22, such that the second extension film layer 322 is coated on the coated area 222 of the second face 22. After the third extension film layer 323 is folded twice and forms a shape in which both sides are folded towards the middle, the third extension film layer 323 is folded towards the second face 22, such that the third extension film layer 323 coats the second coated area 222. It is to be noted that the order of folding the first extension film layer 321, the second extension film layer 322, and the third extension film layer 323 is not specifically limited in the present disclosure, and may be arranged according to specific requirements.

In some embodiments, the first extension film layer 321 and the second extension film layer 322 completely coat the second coated area 222. Exemplarily, the area of the first extension film layer 321 + the area of the second extension film layer 322 > the area of the second coated area 222. Thus, the first extension film layer 321 and the second extension film layer 322 coated on the second coated area 222 have an overlapping area. Alternatively, the area of the first extension film layer 321 + the area of the second extension film layer 322 = the area of the second coated area 222. Thus, the first extension film layer 321 and the second extension film layer 322 completely coat the coated area 222 of the second face 22 and do not overlap.

In some embodiments, the first extension film layer 321 and the second extension film layer 322 do not completely coat the second coated area 222. Exemplarily, the area of the first extension film layer 321 + the area of the second extension film layer 322 < the area of the second coated area 222. Thus, after the first extension film layer 321 and the second extension film layer 322 are folded over the second coated area 222, there is a gap between the first extension film layer 321 and the second extension film layer 322.

Finally, the first extension film layer 321, the second extension film layer 322, and the third extension film layer 323 that coat the second coated area 222 are flattened to form a first layer of the biofilm layer 3 that coats the second face 22.

It is to be noted that in the present disclosure, no matter whether the first extension film layer 321 and the second extension film layer 322 completely coat the second coated area 222, both of them together with the third extension film layer 323 jointly form a layer of the biofilm layer 3 coating the second face 22. That is, in one coating process, the extension film layer 32 forms "one layer" of the biofilm layer 3 coating the second coated area 222. Therefore, upon completion of the coating step from the first face 21 to the second face 22, one layer of the biofilm layer 3 is coated on both the first face 21 and the second face 22 of the mold 2.

S3: coating the biofilm layer 3 on the second coated area 222 of the second face 22, wherein the biofilm layer 3 forms a coating film layer 31 covering the second coated area 222 and an extension film layer 32 extending outward from the second coated area 222; bending the extension film layer 32 in the direction of the first face 21, so that the extension film layer 32 covers the first coated area 212 of the first face 21; and flattening the extension film layer 32 on the first face 21, so that the first face 21 and the second face 22 are coated with two layers of the biofilm layers 3 respectively, thereby completing the coating step in the direction from the second face 22 to the first face 21.

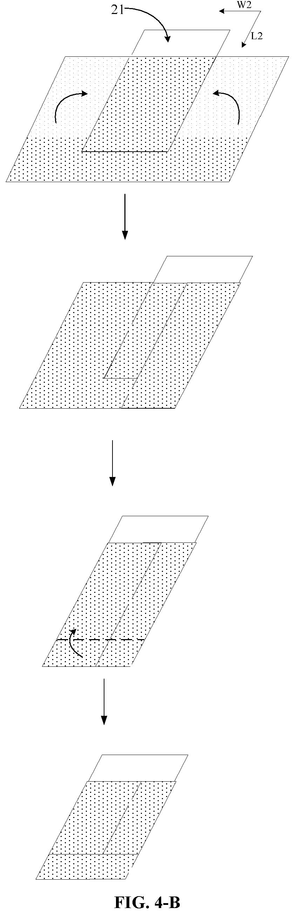

Specifically, as shown in FIG. 4-B, after completion of the coating in the direction from the first face 21 to the second face 22, an additional biofilm layer 3 is taken to coat the second coated area 222, where the portion of the biofilm layer 3 covering the second coated area 222 serves as the coating film layer 31, and the portion connecting the coating film layer 31 and extending outward is the extension film layer 32. The extension film layer 32 includes a first extension film layer 321 and a second extension film layer 322 on two opposite sides in the width direction of the mold 2, and a third extension film layer 323 that connects the first extension film layer 321 and the second extension film layer 322.

The extension film layer 32 is bent in the direction of the first face 21. By folding three times, the first extension film layer 321, the second extension film layer 322, and the third extension film layer 323 are all coated on the first coated area 212. The specific folding process is as shown in S2.

In some embodiments, the first extension film layer 321 and the second extension film layer 322 completely coat the first coated area 212. Exemplarily, the area of the first extension film layer 321 + the area of the second extension film layer 322 > the area of the first coated area 212. Thus, the first extension film layer 321 and the second extension film layer 322 coated on the first coated area 212 have an overlapping area. Alternatively, the area of the first extension film layer 321 + the area of the second extension film layer 322 = the area of the first coated area 212. Thus, the first extension film layer 321 and the second extension film layer 322 completely cover the first coated area 212 and do not overlap.

In some embodiments, the first extension film layer 321 and the second extension film layer 322 do not completely coat the first coated area 212. Exemplarily, the area of the first extension film layer 321 + the area of the second extension film layer 322 < the area of the first coated area 212. Thus, after the first extension film layer 321 and the second extension film layer 322 are folded over the first coated area 212, there is a gap between the first extension film layer 321 and the second extension film layer 322.

Finally, the first extension film layer 321, the second extension film layer 322, and the third extension film layer 323 coated on the first coated area 212 are flattened to form a second layer of the biofilm layer 3 coating the first face 21.

It is to be noted that in the present disclosure, no matter whether the first extension film layer 321 and the second extension film layer 322 completely coat the first coated area 212, both of them together with the third extension film layer 323 jointly form one layer of the biofilm layer 3 coating the first face 21. That is, in one coating process, the extension film layer 32 forms "one layer" of the biofilm layer 3 coating the first coated area 212. Therefore, after completion of the coating step in the direction from the second face 22 to the first face 21, two layers of the biofilm layers 3 are coated on both the first face 21 and the second face 22 of the mold 2.

S4: repeating steps S2 and S3 at least once, such that both the first face 21 and the second face 22 of the mold 2 are coated with at least 4 layers of the biofilm layers 3.

In some embodiments, after completion of the coating step of the biofilm layer 3, the biofilm layers 3 coated on the mold 2 are subjected to freeze-drying treatment in a non-compression environment, such that at least 2 layers of the biofilm layers 3 are integrated to obtain the biological sleeve integrally molded by the transitional connecting portion 14, the first surface body 11, and the second surface body 12.

Furthermore, the plate-shaped mold 2 coated with the biofilm layers 3 is placed in a vacuum freeze dryer for non-compression freeze drying. Preferably, the conditions for the non-compression freeze drying comprise: pre-freezing the plate-shaped mold 2 coated with the biofilm layers 3 to -45°C and holding for 1 to 2 h; then adjusting the temperature to -15°C and holding for 5 to 7 h; readjusting the temperature to 0°C and holding for 2 h; and finally adjusting the temperature to 25°C and holding for 4 h.

The biofilm layer 3 is freeze-dried and molded by the freeze-drying technique under non-compression conditions to obtain the integrally molded biological sleeve with the pocket structure 1.

In some specific embodiments, the size (width × height, unit: cm) of the biological sleeve is 5.4×5; 6.9×6.5; 6.9×8; 6.9×9.5, etc.

In some embodiments, a punching step is further included after the freeze-drying step. Specifically, the biological sleeve is removed from the mold 2 and cut to the desired size. Afterwards, the biological sleeve is put in a mechanical punch, and holes are punched on the first surface body 11 and/or the second surface body 12 to form at least one microhole on the first surface body 11 and/or the second surface body 12.

In some specific embodiments, the first surface body 11 and/or the second surface body 12 are punched with a hole spacing of 10 mm and a hole diameter of 3 mm. Preferably, both the first surface body 11 and the second surface body 12 are provided with a microhole having a hole spacing of 10 mm and a hole diameter of 3 mm.

In some embodiments, a sterilization step is further included after the punching step. Specifically, after heat preservation treatment of the biological sleeve, the biological sleeve is subjected to sterilization treatment with ethylene oxide, and then performing aeration to the ethylene oxide to obtain the sterilized biological sleeve. Preferably, the temperature of the heat preservation treatment is 20°C to 40°C, the time is 2 to 4 hours, and the humidity is 30% to 70%. The concentration of ethylene oxide introduced is 300 to 1000 mg/L, and the time of the sterilization treatment is 4 to 8 hours. In some preferred embodiments, the step of performing aeration to the ethylene oxide is carried out in an aeration chamber with the temperature controlled at 10°C to 30°C for 14 to 28 days.

The sterilization step allows for a biological sleeve that has high biosafety and is suitable for encapsulating the medical device to be implanted *in vivo,* so as to reduce the occurrence of complications such as infection and inflammation after the device is implanted *in vivo.*

In some embodiments, prior to the sterilization step, a gap with a certain length (which is 1/4 to 1/2 of the height of each of various specifications of the product) is cut at one end of the biological sleeve to facilitate the placement and removal of CIED.

The preparation method for the biological sleeve provided in the present disclosure may be used to prepare an integrally molded biological sleeve without suturing the first surface body 11 and the second surface body 12 with sutures, which simplifies the preparation process and reduces the preparation cost of the biological sleeves. Moreover, compared with the sutured biological sleeves, the integrally molded biological sleeves have high tensile strength, may improve the stability in use of the devices after they encapsulate the devices to be implanted *in vivo,* and reduce the probability of complications.

### Examples

The embodiments of the present disclosure will be described in detail below with reference to examples. However, a person skilled in the art would appreciate that the following examples are merely intended to illustrate the present disclosure and shall not be construed as limiting the scope of the present disclosure. Where the specific conditions are not indicated in the examples, conventional conditions or those recommended by manufacturers will be followed. The reagents or instruments used herein, whose manufacturers are not indicated, are all commercially-available conventional products.

### Example 1

The present example provided a method for preparing a small intestinal submucosa material, specifically comprising the following steps:

### (1) Primary treatment of raw material

Porcine small intestinal submucosa tissue was taken and cut to a prescribed size. The lymphoid tissue was removed. The resulting porcine small intestinal submucosa tissue was rinsed until the surface was free of stains, and the water was drained. The prescribed size referred to a width of 2 to 10 cm and a length of 2 to 20 cm.

### (2) Virus inactivation treatment

The porcine small intestinal submucosa tissue was immersed in a virus inactivation solution containing 2% (v/v) peroxyacetic acid and 20% (v/v) ethanol for virus inactivation treatment. The volume ratio of the virus inactivation solution to the porcine small intestinal submucosa tissue was 40:1. The inactivation time was 4 hours. The temperature range was 40°C.

### (3) Primary cleaning

The porcine small intestinal submucosa tissue was cleaned with a cleaning solution until the detected conductivity was 10 µS/cm or less. The cleaning solution was a PBS solution at pH 6-8. The temperature of the cleaning solution was 30°C. The volume ratio of the cleaning solution to the porcine small intestinal submucosa tissue was 40:1. The tissue was cleaned three times, 20 min for each time. Thereafter, the tissue was cleaned with water for injection at 30°C at a volume ratio of the water for injection to the porcine small intestinal submucosa tissue of 40:1 until the detected conductivity was 10 µS/cm or less. The cleaning process was carried out in an ultrasonic cleaner.

### (4) Decellularization treatment

The decellularization solution is a solution using 1 wt% of trypsin solution and 0.2 wt% of EDTA solution, and the solvent used is a PBS solution at pH 6-8. The mixing volume ratio of the decellularization solution to the porcine small intestinal submucosa tissue was 40:1. The decellularization process was carried out in an ultrasonic cleaner at an ultrasonic power of 5000 W or greater. The cleaning time taken in the decellularization process was 30 min at a temperature of 30°C.

### (5) Secondary cleaning

The porcine small intestinal submucosa tissue was cleaned with a cleaning solution until the detected conductivity was 1 µS/cm or less. The cleaning solution was a PBS solution at pH 6-8; the temperature of the cleaning solution was 30°C; and the volume ratio of the cleaning solution to the porcine small intestinal submucosa tissue was 40:1. The tissue was cleaned three times, 20 min for each time. Thereafter, the tissue was cleaned with water for injection at 30°C at a volume ratio of the water for injection to the porcine small intestinal submucosa tissue of 40:1 until the detected conductivity was 1 µS/cm or less, thereby obtaining the sterilized and decellularized small intestinal submucosa material.

### Example 2

The present example provided a method for preparing a biological sleeve, specifically comprising the following steps:

### (1) Fixation molding

The small intestinal submucosa material prepared in Example 1 was used as a biofilm layer. The material was subjected to fixation molding in a mold. The mold was composed of stainless steel baseplates with different sizes (models: 5.4×7; 6.9×8.5; 6.9×10; 6.9×11.5; unit: cm) and a stainless steel blade. The biofilm layer of a certain size (12 cm or 15 cm) was wound around a baseplate, and about 5 mm to about 10 mm of the material were left at the bottom, folded towards one surface, and smoothed with a stainless steel blade to complete the forward coating step. In the opposite direction, a new biofilm layer was wound around a baseplate, and about 5 mm to about 10 mm of the material were left at the bottom, folded towards the other surface, and scraped flat to complete the backward coating step. This process was repeated once, and the material was scraped flat to finally obtain double-sided, 4-layered SIS biological sleeves with the models of (5.4×5; 6.9×6.5; 6.9×8; 6.9×9.5; width × height in cm).

### (2) Vacuum freeze drying

The non-compression freeze drying was carried out in a vacuum freeze dryer. The mold together with the wrapped biofilm layer was pre-frozen at -45°C and held for 1 to 2 hours. Then, the temperature was adjusted to -15°C and held for 5 to 7 hours. The temperature was readjusted to 0°C and held for 2 hours. Finally, the temperature was adjusted to 25°C and held for 4 hours. The vacuum freeze drying was completed.

### (3) Punching and packaging

The details were as follows: the biological sleeve freeze-dried in step (2) was removed from the stainless steel baseplate, cut to a fixed size on a mold, and then put in a mechanical punch for punching at a spacing of 10 mm and a hole diameter of 3 mm.

A gap with a certain length (about 1/2z of the height of each of the specifications of the product) was cut with stainless steel scissors at one end of the biological sleeve to facilitate the placement and removal of CIED, and then the biological sleeve was packaged in a Tyvek^{™} packaging bag.

### (4) Sterilization

The biological sleeves were sterilized with ethylene oxide under the following conditions: holding at 20°C to 40°C for 2 to 4 hours at a humidity of 30% to 70%, and then introducing ethylene oxide at a concentration of 300 to 1000 mg/L, and sterilizing for 4 to 8 hours. The aeration process was carried out in a ventilated aeration chamber with the temperature controlled between 10°C and 30°C for 14 to 28 days.

### Performance Test

Prepared Sample 2: The small intestinal submucosa material prepared in Example 1 was used as a biofilm layer, subjected to vacuum freeze drying, and then sutured using a sewing machine (JH9870, Brother (China) Ltd.) at a needle spacing of 2 mm to prepare a dry-suture SIS biological sleeve.

Prepared Sample 1: The integrated SIS biological sleeve prepared in Example 2 was used as a dry-integrated SIS biological sleeve.

Prepared Sample 4: The small intestinal submucosa material prepared in Example 1 was used as a biofilm layer, subjected to vacuum freeze drying, and then sutured using a sewing machine (JH9870, Brother (China) Ltd.) at a needle spacing of 2 mm. The material was placed in a PBS solution for 2 to 5 min for hydration to prepare a hydration-suture SIS biological sleeve.

Prepared Sample 3: The integrated SIS biological sleeve prepared in Example 2 was placed in a PBS solution for 2 to 5 min for hydration to prepare a hydration-integrated SIS biological sleeve.

Samples 1, 2, 3, and 4 were tested for tensile strength. The materials were tailored to samples with a width of 20 mm and a length of 30 mm. The distance between the fixtures was 15 mm. Samples 1 to 4 were clamped onto the fixtures, respectively. The values of the maximum forces of the samples were measured by a medical packaging tester (model: MED-1, Labthink International, Inc.).

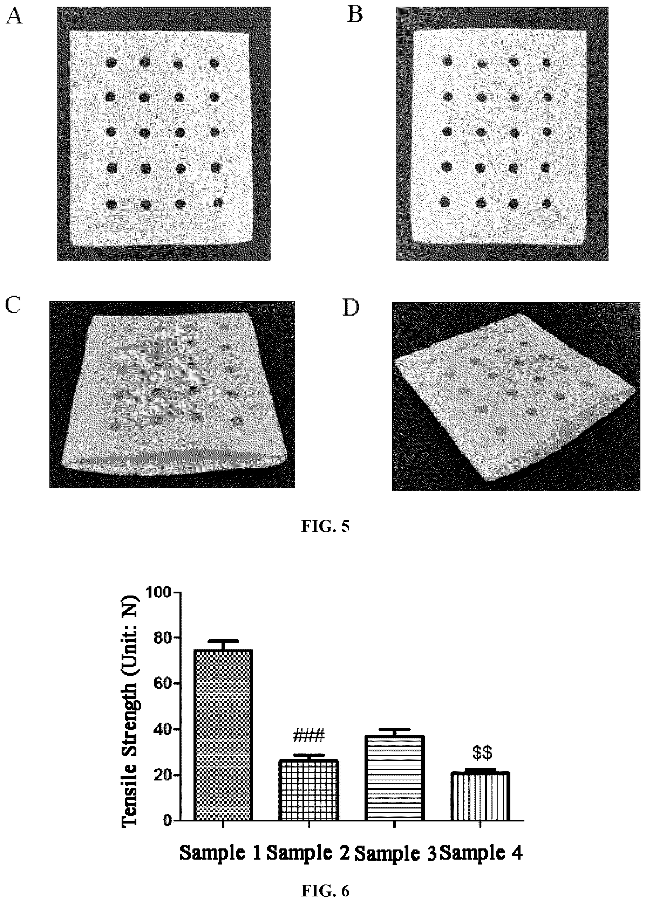

FIG. 6 showed a statistical chart of the tensile strength of the dry-integrated and dry-suture SIS biological sleeves of Sample 1 and Sample 2. There was a ### statistical difference between Sample 1 and Sample 2, and p value < 0.001 indicated that when the biological sleeves were in a dry state, the tensile strength of the sutured biological sleeve was far less than that of the integrated biological sleeve. FIG. 6 also showed a statistical chart of the tensile strength of the hydration-integrated and hydration-suture SIS biological sleeves of Sample 3 and Sample 4. There was a $$ statistical difference between Sample 3 and Sample 4, and p value < 0.01 indicated that after hydration of the biological sleeves, the tensile strength of the sutured biological sleeve was less than that of the integrated biological sleeve. In conclusion, the results suggested that no matter when the SIS biological sleeves are in a dry state or in a hydration state, the tensile strength of the sutured biological sleeve was always less than that of the integrated biological sleeve, which thus indicated that the integrated biological sleeves had better tensile strength after implanted *in vivo.*

## Claims

1. A biological sleeve, being formed of a sterilized and decellularized extracellular matrix material, and having an integrally molded pocket structure; said pocket structure having an accommodation cavity for accommodating an implantable medical device, and an opening (13) that communicates the accommodation cavity with the outside,
the accommodation cavity being enclosed by a first surface body (11) and a second surface body (12) that are opposite to each other of said decellularized extracellular matrix; a portion of said first surface body and of said second surface body being connected to each other thereby forming a transitional connecting portion (14) of the biological sleeve;
wherein
- the biological sleeve is prepared by subjecting a plate-shaped mold (2) previously coated with at least two biofilm layers to freeze drying in a non-compression environment, so as to integrate the at least two layers of the biofilm; wherein said at least two biofilm layers coating a first face (21) and a second face (22) of the mold (2) form the first surface body (11) and the second surface body (12) respectively of the biological sleeve, in which the at least two layers of the biofilm connecting the first surface body (11) and the second surface body (12) forms a transitional connecting portion (14), said at least two biofilm layers being made of the decellularized extracellular matrix material;
said previous coating on the mold with at least two layers of the decellularized extracellular matrix material comprising the following steps:
• taking a plate-shaped mold (2) with both sides being a first face (21) and a second face (22) respectively, and reserving top areas (211) and (221) of the first face (21) and of the second face (22) that are opposite to each other as non-coated areas along the length direction of the mold, and the remaining areas as coated areas;
• coating with a first biofilm layer (3) on the first face (21) of the mold (2), wherein the first biofilm layer (3) is formed by the coating film layer (31) covering the first face (21) and an extension film layer (32) of the first biofilm layer (3) extending outward from the coated area ;
• bending the extension film layer(32) in the direction of the second face (22) of the mold (2), so that the extension film layer (32) covers the second face (22) of the mold (2) ; and flattening the extension film layer (32) on the second face (22) of the mold to complete the coating step in the direction from the first face to the second face;
• coating with a second biofilm layer (3) on the second face (22) of the mold (2), said second biofilm layer being formed by the coating film layer (31) covering the second face (22) and an extension film layer (32) extending outward from the coated area;
• bending the extension film layer (32) of the second biofilm layer (3) in the direction of the first face (21), so that the extension film layer (32) of the second biofilm layer (3) covers the coated area by first film layer (3) of the first face (21) of the mold (2); and
• flattening the second extension film layer (32) on the first face (21) to complete the coating step in the direction from the second face (22) to the first face (21).

2. The biological sleeve according to claim 1, wherein the transitional connecting portion has a length of 5 to 10 cm along the length direction of the biological sleeve, and the transitional connecting portion has a length of 4 to 8 cm along the width direction of the biological sleeve.

3. The biological sleeve according to claim 2, wherein at least one microhole is provided on the first surface body and/or the second surface body; optionally, the microhole has a diameter of 1 to 3 mm; optionally, the spacing between the microholes is 10 to 15 mm.

4. The biological sleeve according to any one of claims 1 to 3, wherein the sterilized and decellularized extracellular matrix material is obtained by taking small intestinal submucosa tissue and subjecting it to virus inactivation treatment and decellularization treatment;
preferably, a step of the virus inactivation treatment comprises immersing the small intestinal submucosa tissue in a virus inactivation solution containing 0.1 to 5% (v/v) peroxyacetic acid and 5 to 40% (v/v) ethanol, and treating for 2 to 4 hours at a temperature of 10°C to 40°C;
preferably, a step of the decellularization treatment comprises: immersing the small intestinal submucosa tissue in a decellularization solution containing 0.1 to 2 wt% of trypsin and 0.01 to 0.3 wt% of EDTA, and treating for 10 to 60 min at a temperature of 10°C to 40°C under ultrasonic conditions with ultrasonic power of 5000 W or greater.

5. The biological sleeve according to claim 4, wherein a step is further included between the step of the virus inactivation treatment and the step of the decellularization treatment, the step being a step of cleaning the small intestinal submucosa tissue until detected conductivity of the small intestinal submucosa tissue is reduced to 10 µS/cm or less;
preferably, a step is further included after the step of the decellularization treatment, the step being a step of cleaning the small intestinal submucosa tissue until the detected conductivity of the small intestinal submucosa tissue is reduced to 1 µS/cm or less.

6. A method for preparing a biological sleeve according to any one of claims 1 to 5, wherein the preparation method comprises the following steps:
a preparation step of a biofilm layer: preparing a sterilized and decellularized small intestinal submucosa material as a biofilm layer for preparing the biological sleeve;
a coating step of the biofilm layer: alternately coating the biofilm layers on surfaces of both sides of a plate-shaped mold, so that the surfaces of both sides are coated with at least two layers of the biofilm layers, respectively; wherein any one layer of the biofilm layers comprises a coating film layer coated on a partial area of the surface of one side, and an extension film layer that extends continuously to the surface of the other side corresponding to the partial area; and
a freeze-drying step: subjecting the biofilm layer coated on the mold to a freeze-drying treatment in a non-compression environment, so as to integrate the at least two layers of the biofilm layers; wherein the biofilm layers coating two surfaces form a first surface body and a second surface body respectively, the biofilm layer connecting the first surface body and the second surface body forms a transitional connecting portion, and the side of the biofilm layer that does not coat the mold forms an opening, thereby obtaining a biological sleeve integrally molded by the first surface body, the second surface body, and the transitional connecting portion.

7. The preparation method according to claim 6,
in the coating step in the direction from the first face to the second face, the extension film layer of the first biofilm layer completely coats the coated area of the second face; and/or in the coating step in the direction from the first face to the second face, the extension film layer of the second biofilm layer completely coats the coated area of the first face;
preferably, the coating step in the direction from the first face to the second face, and the coating step in the direction from the first face to the second face are repeated at least once.

8. The preparation method according to claim 6, wherein the step of the freeze-drying treatment comprises: placing the plate-shaped mold coated with the biofilm layers in a vacuum freeze dryer for non-compression freeze drying;
preferably, conditions for the non-compression freeze drying comprise: pre-freezing to -45°C and holding for 1 to 2 h; then adjusting the temperature to -15°C and holding for 5 to 7 h; readjusting the temperature to 0°C and holding for 2 h; and finally adjusting the temperature to 25°C and holding for 4 h.

9. The preparation method according to any one of claims 6 to 8, wherein the preparation method further comprises the following steps:
a punching step: removing the biological sleeve from the mold, cutting to a desired size, and then punching a hole in the first surface body and/or the second surface body to form at least one microhole on the first surface body and/or the second surface body; preferably, punching a hole in the first surface body and/or the second surface body with microhole spacing of 10 to 15 mm and the diameter of 1 to 3 mm; and
a sterilization step: subjecting the biological sleeve to sterilization treatment with ethylene oxide after heat preservation treatment of the biological sleeve, and then performing aeration to the ethylene oxide to obtain a sterilized biological sleeve; preferably, the heat preservation treatment is carried out for 2 to 4 hours at a temperature of 20°C to 40°C and a humidity of 30% to 70%; preferably, the ethylene oxide is introduced at a concentration of 300 to 1000 mg/L, and the time of the sterilization treatment is 4 to 8 h; preferably, the step of performing aeration to the ethylene oxide is carried out in a ventilated aeration chamber with the temperature controlled at 10°C to 30°C for 14 to 28 days.

10. An implantable medical apparatus, wherein the implantable medical apparatus comprises:
a biological sleeve according to any one of claims 1 to 5, or a biological sleeve prepared by a method according to any one of claims 6 to 9; or
an implantable medical device which comprises: a biological sleeve according to any one of claims 1 to 5 or a biological sleeve prepared by a method according to any one of claims 6 to 9; said implantable medical device being at least partially placed in an accommodation cavity of the biological sleeve.

11. Implantable medical device according to claim 10, wherein the implantable medical device is anyone selected from the group consisting of: a cardiac pacemaker, an implantable cardioverter defibrillator, a cardiac resynchronization therapy pacemaker, an implantable defibrillator, an insertable cardiac monitor, and an implantable cardiovascular monitor.

12. Use of a biological sleeve according to any one of claims 1 to 5 or a biological sleeve prepared by a method according to any one of claims 6 to 9 in accommodation of an implantable medical device;

13. The implantable medical device according to any one of claims 10 or 11 for use in diagnosis, monitoring and/or treatment of cardiovascular diseases.

## Patentansprüche

1. Biologische Hülse, die aus einem sterilisierten und dezellularisierten extrazellulären Matrixmaterial gebildet ist und eine einstückig geformte Taschenstruktur aufweist; wobei die Taschenstruktur einen Aufnahmehohlraum zur Aufnahme einer implantierbaren medizinischen Vorrichtung und eine Öffnung (13) aufweist, die den Aufnahmehohlraum mit der Außenseite verbindet,
wobei der Aufnahmehohlraum von einem ersten Oberflächenkörper (11) und einem zweiten Oberflächenkörper (12) umschlossen ist, die einander gegenüberliegen, aus der dezellularisierten extrazellulären Matrix; wobei ein Abschnitt des ersten Oberflächenkörpers und des zweiten Oberflächenkörpers miteinander verbunden ist, wodurch ein Übergangsverbindungsabschnitt (14) der biologischen Hülse gebildet wird;
wobei
- die biologische Hülse durch Unterziehen einer plattenförmigen Form (2), die zuvor mit mindestens zwei Biofilmschichten beschichtet wurde, einer Gefriertrocknung in einer kompressionsfreien Umgebung hergestellt wird, um die mindestens zwei Schichten des Biofilms zu integrieren; wobei die mindestens zwei Biofilmschichten, die eine erste Fläche (21) und eine zweite Fläche (22) der Form (2) beschichten, den ersten Oberflächenkörper (11) bzw. den zweiten Oberflächenkörper (12) der biologischen Hülse bilden, wobei die mindestens zwei Schichten des Biofilms, die den ersten Oberflächenkörper (11) und den zweiten Oberflächenkörper (12) verbinden, einen Übergangsverbindungsabschnitt (14) bilden, wobei die mindestens zwei Biofilmschichten aus dem dezellularisierten extrazellulären Matrixmaterial hergestellt sind;
wobei das vorherige Beschichten der Form mit mindestens zwei Schichten des dezellularisierten extrazellulären Matrixmaterials die folgenden Schritte umfasst:
• Nehmen einer plattenförmigen Form (2), deren beide Seiten eine erste Fläche (21) bzw. eine zweite Fläche (22) sind, und Freilassen von oberen Bereichen (211) und (221) der ersten Fläche (21) und der zweiten Fläche (22), die einander gegenüberliegen, als unbeschichtete Bereiche entlang der Längsrichtung der Form, und der verbleibenden Bereiche als beschichtete Bereiche;
• Beschichten mit einer ersten Biofilmschicht (3) auf der ersten Fläche (21) der Form (2), wobei die erste Biofilmschicht (3) durch die Beschichtungsfilmschicht (31), die die erste Fläche (21) bedeckt, und eine Verlängerungsfilmschicht (32) der ersten Biofilmschicht (3), die sich von dem beschichteten Bereich nach außen erstreckt, gebildet wird;
• Biegen der Verlängerungsfilmschicht (32) in Richtung der zweiten Fläche (22) der Form (2), so dass die Verlängerungsfilmschicht (32) die zweite Fläche (22) der Form (2) bedeckt; und Glätten der Verlängerungsfilmschicht (32) auf der zweiten Fläche (22) der Form, um den Beschichtungsschritt in der Richtung von der ersten Fläche zur zweiten Fläche abzuschließen;
• Beschichten mit einer zweiten Biofilmschicht (3) auf der zweiten Fläche (22) der Form (2), wobei die zweite Biofilmschicht durch die Beschichtungsfilmschicht (31), die die zweite Fläche (22) bedeckt, und eine Verlängerungsfilmschicht (32), die sich von dem beschichteten Bereich nach außen erstreckt, gebildet wird;
• Biegen der Verlängerungsfilmschicht (32) der zweiten Biofilmschicht (3) in Richtung der ersten Fläche (21), so dass die Verlängerungsfilmschicht (32) der zweiten Biofilmschicht (3) den durch die erste Filmschicht (3) der ersten Fläche (21) der Form (2) beschichteten Bereich bedeckt; und
• Glätten der zweiten Verlängerungsfilmschicht (32) auf der ersten Fläche (21), um den Beschichtungsschritt in der Richtung von der zweiten Fläche (22) zur ersten Fläche (21) abzuschließen.

2. Biologische Hülse nach Anspruch 1, wobei der Übergangsverbindungsabschnitt eine Länge von 5 bis 10 cm entlang der Längsrichtung der biologischen Hülse aufweist, und der Übergangsverbindungsabschnitt eine Länge von 4 bis 8 cm entlang der Breitenrichtung der biologischen Hülse aufweist.

3. Biologische Hülse nach Anspruch 2, wobei mindestens ein Mikroloch auf dem ersten Oberflächenkörper und/oder dem zweiten Oberflächenkörper vorgesehen ist; optional das Mikroloch einen Durchmesser von 1 bis 3 mm aufweist; optional der Abstand zwischen den Mikrolöchern 10 bis 15 mm beträgt.

4. Biologische Hülse nach einem der Ansprüche 1 bis 3, wobei das sterilisierte und dezellularisierte extrazelluläre Matrixmaterial durch Entnahme von Dünndarm-Submukosa-Gewebe und dessen Unterziehen einer Virusinaktivierungsbehandlung und einer Dezellularisierungsbehandlung erhalten wird;
vorzugsweise ein Schritt der Virusinaktivierungsbehandlung das Eintauchen des Dünndarm-Submukosa-Gewebes in eine Virusinaktivierungslösung, die 0,1 bis 5 % (v/v) Peroxyessigsäure und 5 bis 40 % (v/v) Ethanol enthält, und das Behandeln für 2 bis 4 Stunden bei einer Temperatur von 10 °C bis 40 °C umfasst;
vorzugsweise ein Schritt der Dezellularisierungsbehandlung Folgendes umfasst: Eintauchen des Dünndarm-Submukosa-Gewebes in eine Dezellularisierungslösung, die 0,1 bis 2 Gew.-% Trypsin und 0,01 bis 0,3 Gew.-% EDTA enthält, und das Behandeln für 10 bis 60 min bei einer Temperatur von 10 °C bis 40 °C unter Ultraschallbedingungen mit einer Ultraschallleistung von 5000 W oder mehr.

5. Biologische Hülse nach Anspruch 4, wobei ein Schritt ferner zwischen dem Schritt der Virusinaktivierungsbehandlung und dem Schritt der Dezellularisierungsbehandlung eingeschlossen ist, wobei der Schritt ein Schritt des Reinigens des Dünndarm-Submukosa-Gewebes ist, bis die erfasste Leitfähigkeit des Dünndarm-Submukosa-Gewebes auf 10 µS/cm oder weniger reduziert ist;
vorzugsweise ein Schritt ferner nach dem Schritt der Dezellularisierungsbehandlung eingeschlossen ist, wobei der Schritt ein Schritt des Reinigens des Dünndarm-Submukosa-Gewebes ist, bis die erfasste Leitfähigkeit des Dünndarm-Submukosa-Gewebes auf 1 µS/cm oder weniger reduziert ist.

6. Verfahren zur Herstellung einer biologischen Hülse nach einem der Ansprüche 1 bis 5, wobei das Herstellungsverfahren die folgenden Schritte umfasst:
einen Herstellungsschritt einer Biofilmschicht: Herstellen eines sterilisierten und dezellularisierten Dünndarm-Submukosa-Materials als Biofilmschicht zur Herstellung der biologischen Hülse;
einen Beschichtungsschritt der Biofilmschicht: abwechselndes Beschichten der Biofilmschichten auf Oberflächen beider Seiten einer plattenförmigen Form, so dass die Oberflächen beider Seiten jeweils mit mindestens zwei Schichten der Biofilmschichten beschichtet werden; wobei eine beliebige Schicht der Biofilmschichten eine Beschichtungsfilmschicht, die auf einen Teilbereich der Oberfläche einer Seite aufgetragen ist, und eine Verlängerungsfilmschicht umfasst, die sich kontinuierlich zu der Oberfläche der anderen Seite entsprechend dem Teilbereich erstreckt; und
einen Gefriertrocknungsschritt: Unterziehen der auf die Form aufgetragenen Biofilmschicht einer Gefriertrocknungsbehandlung in einer kompressionsfreien Umgebung, um die mindestens zwei Schichten der Biofilmschichten zu integrieren; wobei die Biofilmschichten, die zwei Oberflächen beschichten, jeweils einen ersten Oberflächenkörper und einen zweiten Oberflächenkörper bilden, die Biofilmschicht, die den ersten Oberflächenkörper und den zweiten Oberflächenkörper verbindet, einen Übergangsverbindungsabschnitt bildet, und die Seite der Biofilmschicht, die die Form nicht beschichtet, eine Öffnung bildet, wodurch eine biologische Hülse erhalten wird, die durch den ersten Oberflächenkörper, den zweiten Oberflächenkörper und den Übergangsverbindungsabschnitt einstückig geformt ist.

7. Herstellungsverfahren nach Anspruch 6,
wobei in dem Beschichtungsschritt in der Richtung von der ersten Fläche zur zweiten Fläche die Verlängerungsfilmschicht der ersten Biofilmschicht den beschichteten Bereich der zweiten Fläche vollständig beschichtet; und/oder in dem Beschichtungsschritt in der Richtung von der zweiten Fläche zur ersten Fläche die Verlängerungsfilmschicht der zweiten Biofilmschicht den beschichteten Bereich der ersten Fläche vollständig beschichtet;
vorzugsweise der Beschichtungsschritt in der Richtung von der ersten Fläche zur zweiten Fläche und der Beschichtungsschritt in der Richtung von der zweiten Fläche zur ersten Fläche mindestens einmal wiederholt werden.

8. Herstellungsverfahren nach Anspruch 6, wobei der Schritt der Gefriertrocknungsbehandlung Folgendes umfasst: Platzieren der mit den Biofilmschichten beschichteten plattenförmigen Formin einem Vakuum-Gefriertrockner zur kompressionsfreien Gefriertrocknung;
vorzugsweise die Bedingungen für die kompressionsfreie Gefriertrocknung Folgendes umfassen: Vorgefrieren auf -45 °C und Halten für 1 bis 2 h; dann Einstellen der Temperatur auf -15 °C und Halten für 5 bis 7 h; erneutes Einstellen der Temperatur auf 0 °C und Halten für 2 h; und schließlich Einstellen der Temperatur auf 25 °C und Halten für 4 h.

9. Herstellungsverfahren nach einem der Ansprüche 6 bis 8, wobei das Herstellungsverfahren ferner die folgenden Schritte umfasst:
einen Stanzschritt: Entfernen der biologischen Hülse von der Form, Zuschneiden auf eine gewünschte Größe und dann Stanzen eines Lochs in den ersten Oberflächenkörper und/oder den zweiten Oberflächenkörper, um mindestens ein Mikroloch auf dem ersten Oberflächenkörper und/oder dem zweiten Oberflächenkörper zu bilden; vorzugsweise Stanzen eines Lochs in den ersten Oberflächenkörper und/oder den zweiten Oberflächenkörper mit einem Mikrolochabstand von 10 bis 15 mm und einem Durchmesser von 1 bis 3 mm; und
einen Sterilisationsschritt: Unterziehen der biologischen Hülse einer Sterilisationsbehandlung mit Ethylenoxid nach einer Wärmekonservierungsbehandlung der biologischen Hülse, und dann Durchführen einer Belüftung des Ethylenoxids, um eine sterilisierte biologische Hülse zu erhalten; vorzugsweise die Wärmekonservierungsbehandlung für 2 bis 4 Stunden bei einer Temperatur von 20 °C bis 40 °C und einer Luftfeuchtigkeit von 30 % bis 70 % durchgeführt wird; vorzugsweise das Ethylenoxid in einer Konzentration von 300 bis 1000 mg/L eingeführt wird und die Zeit der Sterilisationsbehandlung 4 bis 8 h beträgt; vorzugsweise der Schritt der Durchführung der Belüftung des Ethylenoxids in einer belüfteten Belüftungskammer bei einer kontrollierten Temperatur von 10 °C bis 30 °C für 14 bis 28 Tage durchgeführt wird.

10. Implantierbare medizinische Vorrichtung, wobei die implantierbare medizinische Vorrichtung Folgendes umfasst:
eine biologische Hülse nach einem der Ansprüche 1 bis 5, oder eine biologische Hülse, die durch ein Verfahren nach einem der Ansprüche 6 bis 9 hergestellt wurde; und
eine implantierbare medizinische Vorrichtung, die zumindest teilweise in einem Aufnahmehohlraum der biologischen Hülse platziert ist.

11. Implantierbare medizinische Vorrichtung nach Anspruch 10, wobei die implantierbare medizinische Vorrichtung eine beliebige ist, die aus der Gruppe ausgewählt ist, die aus Folgendem besteht: einem Herzschrittmacher, einem implantierbaren Kardioverter-Defibrillator, einem Herzschrittmacher für die kardiale Resynchronisationstherapie, einem implantierbaren Defibrillator, einem einsetzbaren Herzmonitor und einem implantierbaren kardiovaskulären Monitor.

12. Verwendung einer biologischen Hülse nach einem der Ansprüche 1 bis 5 oder einer biologischen Hülse, die durch ein Verfahren nach einem der Ansprüche 6 bis 9 hergestellt wurde, zur Aufnahme einer implantierbaren medizinischen Vorrichtung.

13. Implantierbare medizinische Vorrichtung nach einem der Ansprüche 10 oder 11 zur Verwendung bei der Diagnose, Überwachung und/oder Behandlung von Herz-Kreislauf-Erkrankungen.

## Revendications

1. Manchon biologique, étant constitué d'un matériau de matrice extracellulaire décellularisé et stérilisé, et présentant une structure de poche moulée d'un seul tenant; ladite structure de poche présentant une cavité de logement pour loger un dispositif médical implantable, et une ouverture (13) qui fait communiquer la cavité de logement avec l'extérieur,
la cavité de logement étant délimitée par un premier corps de surface (11) et un second corps de surface (12) qui sont opposés l'un à l'autre dudit matériau de matrice extracellulaire décellularisé ; une partie dudit premier corps de surface et dudit second corps de surface étant reliée l'une à l'autre, formant ainsi une partie de liaison de transition (14) du manchon biologique;
dans lequel
- le manchon biologique est préparé en soumettant un moule en forme de plaque (2) préalablement revêtu d'au moins deux couches de biofilm à une lyophilisation dans un environnement sans compression, de manière à intégrer les au moins deux couches du biofilm ; dans lequel lesdites au moins deux couches de biofilm revêtant une première face (21) et une seconde face (22) dumoule (2) forment respectivement le premier corps de surface (11) et le second corps de surface (12) du manchon biologique, dans lequel les au moins deux couches du biofilm reliant le premier corps de surface (11) et le second corps de surface (12) forment une partie de liaison de transition (14), lesdites au moins deux couches de biofilm étant constituées du matériau de matrice extracellulaire décellularisé;
ledit revêtement préalable sur le moule avec au moins deux couches du matériau de matrice extracellulaire décellularisé comprenant les étapes suivantes :
• la prise d'un moule en forme de plaque (2) avec les deux côtés étant respectivement une première face (21) et une seconde face (22), et la réservation de zones supérieures (211) et (221) de la première face (21) et de la seconde face (22) qui sont opposées l'une à l'autre comme zones non revêtues le long de la direction de la longueur du moule, et les zones restantes comme zones revêtues ;
• le revêtement avec une première couche de biofilm (3) sur la première face (21) du moule (2), dans lequel la première couche de biofilm (3) est formée par la couche de film de revêtement (31) recouvrant la première face (21) et une couche de film d'extension (32) de la première couche de biofilm (3) s'étendant vers l'extérieur depuis la zone revêtue ;
• le pliage de la couche de film d'extension (32) en direction de la seconde face (22) du moule (2), de sorte que la couche de film d'extension (32) recouvre la seconde face (22) du moule (2) ; et l'aplatissement de la couche de film d'extension (32) sur la seconde face (22) du moule pour achever l'étape de revêtement dans la direction allant de la première face à la seconde face ;
• le revêtement avec une seconde couche de biofilm (3) sur la seconde face (22) du moule (2), ladite seconde couche de biofilm étant formée par la couche de film de revêtement (31) recouvrant la seconde face (22) et une couche de film d'extension (32) s'étendant vers l'extérieur depuis la zone revêtue ;
• le pliage de la couche de film d'extension (32) de la seconde couche de biofilm (3) en direction de la première face (21), de sorte que la couche de film d'extension (32) de la seconde couche de biofilm (3) recouvre la zone revêtue par la première couche de film (3) de la première face (21) du moule (2) ; et
• l'aplatissement de la seconde couche de film d'extension (32) sur la première face (21) pour achever l'étape de revêtement dans la direction allant de la seconde face (22) à la première face (21).

2. Manchon biologique selon la revendication 1, dans lequel la partie de liaison de transition a une longueur de 5 à 10 cm le long de la direction de la longueur du manchon biologique, et la partie de liaison de transition a une longueur de 4 à 8 cm le long de la direction de la largeur du manchon biologique.

3. Manchon biologique selon la revendication 2, dans lequel au moins un micro-trou est ménagé sur le premier corps de surface et/ou le second corps de surface ; optionnellement, le micro-trou a un diamètre de 1 à 3 mm ; optionnellement, l'espacement entre les micro-trous est de 10 à 15 mm.

4. Manchon biologique selon l'une quelconque des revendications 1 à 3, dans lequel le matériau de matrice extracellulaire décellularisé et stérilisé est obtenu en prenant du tissu de la sous-muqueuse de l'intestin grêle et en le soumettant à un traitement d'inactivation virale et à un traitement de décellularisation ;
de préférence, une étape du traitement d'inactivation virale comprend l'immersion du tissu de la sous-muqueuse de l'intestin grêle dans une solution d'inactivation virale contenant de 0,1 à 5 % (v/v) d'acide peroxyacétique et de 5 à 40 % (v/v) d'éthanol, et le traitement pendant 2 à 4 heures à une température de 10 °C à 40 °C ;
de préférence, une étape du traitement de décellularisation comprend : l'immersion du tissu de la sous-muqueuse de l'intestin grêle dans une solution de décellularisation contenant de 0,1 à 2 % en poids de trypsine et de 0,01 à 0,3 % en poids d'EDTA, et le traitement pendant 10 à 60 min à une température de 10 °C à 40 °C dans des conditions ultrasoniques avec une puissance ultrasonique de 5000 W ou plus.

5. Manchon biologique selon la revendication 4, dans lequel une étape est en outre incluse entre l'étape du traitement d'inactivation virale et l'étape du traitement de décellularisation, l'étape étant une étape de nettoyage du tissu de la sous-muqueuse de l'intestin grêle jusqu'à ce que la conductivité détectée du tissu de la sous-muqueuse de l'intestin grêle soit réduite à 10 µS/cm ou moins ;
de préférence, une étape est en outre incluse après l'étape du traitement de décellularisation, l'étape étant une étape de nettoyage du tissu de la sous-muqueuse de l'intestin grêle jusqu'à ce que la conductivité détectée du tissu de la sous-muqueuse de l'intestin grêle soit réduite à 1 µS/cm ou moins.

6. Procédé de préparation d'un manchon biologique selon l'une quelconque des revendications 1 à 5, dans lequel le procédé de préparation comprend les étapes suivantes :
une étape de préparation d'une couche de biofilm : la préparation d'un matériau de sous-muqueuse de l'intestin grêle décellularisé et stérilisé en tant que couche de biofilm pour la préparation du manchon biologique ;
une étape de revêtement de la couche de biofilm : le revêtement alterné des couches de biofilm sur les surfaces des deux côtés d'un moule en forme de plaque, de sorte que les surfaces des deux côtés sont revêtues d'au moins deux couches des couches de biofilm, respectivement ; dans lequel n'importe quelle couche des couches de biofilm comprend une couche defilm de revêtement revêtue sur une zone partielle de la surface d'un côté, et une couche de film d'extension qui s'étend en continu jusqu'à la surface de l'autre côté correspondant à la zone partielle ; et
une étape de lyophilisation : la soumission de la couche de biofilm revêtue sur le moule à un traitement de lyophilisation dans un environnement sans compression, de manière à intégrer les au moins deux couches des couches de biofilm ; dans lequel les couches de biofilm revêtant deux surfaces forment respectivement un premier corps de surface et un second corps de surface, la couche de biofilm reliant le premier corps de surface et le second corps de surface forme une partie de liaison de transition, et le côté de la couche de biofilm qui ne revêt pas le moule forme une ouverture, obtenant ainsi un manchon biologique moulé d'un seul tenant par le premier corps de surface, le second corps de surface, et la partie de liaison de transition.

7. Procédé de préparation selon la revendication 6,
dans l'étape de revêtement dans la direction allant de la première face à la seconde face, la couche de film d'extension de la première couche de biofilm recouvre complètement la zone revêtue de la seconde face ; et/ou dans l'étape de revêtement dans la direction allant de la seconde face à la première face, la couche de film d'extension de la seconde couche de biofilm recouvre complètement la zone revêtue de la première face ;
de préférence, l'étape de revêtement dans la direction allant de la première face à la seconde face, et l'étape de revêtement dans la direction allant de la seconde face à la première face sont répétées au moins une fois.

8. Procédé de préparation selon la revendication 6, dans lequel l'étape du traitement de lyophilisation comprend : le placement du moule en forme de plaque revêtu avec les couches de biofilm dans un lyophilisateur sous vide pour une lyophilisation sans compression ;
de préférence, les conditions pour la lyophilisation sans compression comprennent: la pré-congélation à -45 °C et le maintien pendant 1 à 2 h ; puis l'ajustement de la température à -15 °C et le maintien pendant 5 à 7 h ; le réajustement de la température à 0 °C et le maintien pendant 2 h ; et enfin l'ajustement de la température à 25 °C et le maintien pendant 4 h.

9. Procédé de préparation selon l'une quelconque des revendications 6 à 8, dans lequel le procédé de préparation comprend en outre les étapes suivantes :
une étape de poinçonnage : le retrait du manchon biologique du moule, la découpe à une taille souhaitée, puis le poinçonnage d'un trou dans le premier corps de surface et/ou le second corps de surface pour former au moins un micro-trou sur le premier corps de surface et/ou le second corps de surface ; de préférence, le poinçonnage d'un trou dans le premier corps de surface et/ou le second corps de surface avec un espacement de micro-trous de 10 à 15 mm et un diamètre de 1 à 3 mm ; et
une étape de stérilisation : la soumission du manchon biologique à un traitement de stérilisation à l'oxyde d'éthylène après un traitement de conservation thermique du manchon biologique, puis la réalisation d'une aération de l'oxyde d'éthylène pour obtenir un manchon biologique stérilisé ; de préférence, le traitement de conservation thermique est effectué pendant 2 à 4 heures à une température de 20 °C à 40 °C et une humidité de 30 % à 70 % ; de préférence, l'oxyde d'éthylène est introduit à une concentration de 300 à 1000 mg/L, et la durée du traitement de stérilisation est de 4 à 8 h ; de préférence, l'étape de réalisation de l'aération de l'oxyde d'éthylène est effectuée dans une chambre d'aération ventilée avec une température contrôlée de 10 °C à 30 °C pendant 14 à 28 jours.

10. Appareil médical implantable, dans lequel l'appareil médical implantable comprend :
un manchon biologique selon l'une quelconque des revendications 1 à 5, ou un manchon biologique préparé par un procédé selon l'une quelconque des revendications 6 à 9 ; et
un dispositif médical implantable qui est au moins partiellement placé dans une cavité de logement du manchon biologique.

11. Appareil médical implantable selon la revendication 10, dans lequel le dispositif médical implantable est l'un quelconque choisi dans le groupe constitué par : un stimulateur cardiaque, un défibrillateur cardioverteur implantable, un stimulateur cardiaque de thérapie de resynchronisation cardiaque, un défibrillateur implantable, un moniteur cardiaque insérable, et un moniteur cardiovasculaire implantable.

12. Utilisation d'un manchon biologique selon l'une quelconque des revendications 1 à 5 ou d'un manchon biologique préparé par un procédé selon l'une quelconque des revendications 6 à 9 pour le logement d'un dispositif médical implantable.

13. Appareil médical implantable selon l'une quelconque des revendications 10 ou 11 pour son utilisation dans le diagnostic, la surveillance et/ou le traitement de maladies cardiovasculaires.
